# EUROPEAN PATENT APPLICATION

(11) **EP 4 635 953 A1**
(43) Date of publication of application: **22.10.2025**
(21) Application number: 22968173.9
(22) Date of filing: 15.12.2022
(51) Int. Cl.: C07D 471/04, A61K 31/437, A61K 31/444, A61P 35/00, A61P 3/10, A61P 3/04, A61P 9/00, A61P 9/10, A61P 25/28, A61P 25/16, A61P 21/00, A61P 25/00, A61P 19/02, A61P 17/02, A61P 17/14, A61P 27/16, A61P 27/02, A61P 1/02, A61P 37/06

(54) **PYRROLOPYRIDINE DERIVATIVE, AND PHARMACEUTICAL COMPOSITION THEREOF AND USE THEREOF**

(71) Applicant: Iregene Therapeutics Co., Ltd, Chengdu, Sichuan 610200 (CN)
(72) Inventor: WEI, Jun, Wuhan, Hubei 430000 (CN); CAI, Meng, Wuhan, Hubei 430000 (CN); WEI, Mengya, Wuhan, Hubei 430000 (CN); CHEN, Xiping, Wuhan, Hubei 430000 (CN)
(74) Representative: Metida
(86) International application number: PCT/CN2022/139170
(87) International publication number: WO 2024/124463

(57) **Abstract**

The present invention relates to a pyrrolopyridine derivative, and a pharmaceutical composition thereof and the use thereof. The pyrrolopyridine derivative is a highly selective activator that can be used for the expression of Oct4 and downstream genes, and has the following formula: (I), wherein m1, m2, A₂ and A₃ are as described herein.

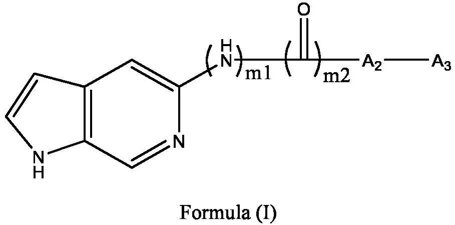

## Description

### Background of the Invention

### Field of the Invention

The present invention relates to the field of medicine, specifically to a pyrrolopyridine derivative and its pharmaceutical composition, as well as its application in the regulation of Oct4 and Oct4-associated genes.

### Description of Related Art

Regenerative medicine refers to the emerging science that uses various new technologies to rebuild aging or functionally impaired tissues and organs, and to treat related diseases through various medical approaches. The important research directions in regenerative medicine include the mechanisms of normal tissue characteristics and functions, the biological basis of post-traumatic repair, as well as the regeneration mechanisms of tissues and organs and the differentiation mechanisms of various stem cells, ultimately aiming to obtain effective biological therapeutic methods. Among these, embryonic stem cells (ESCs) have been the most prominent cell type in early regenerative medicine research. However, obtaining and using these cells involves significant ethical controversies, as research on embryonic stem cells requires the destruction of embryos, which are considered a life form before the human body fully develops. This ethical issue greatly hindered the progress and application of regenerative medicine.

In 2006, the team led by Shinya Yamanaka proposed a "cocktail" method consisting of four transcription factors: Oct4, Sox2, Klf4, and c-Myc, which successfully reprogrammed terminally differentiated skin fibroblasts into pluripotent stem cells. These stem cells, known as induced pluripotent stem cells (iPSCs), possess similar differentiation potential to embryonic stem cells, capable of forming the three primary germ layers of the human body: ectoderm, mesoderm, and endoderm, ultimately forming various adult cells. The introduction of this method overcame the ethical limitations of using human embryonic stem cells in medicine and greatly expanded the potential clinical applications of stem cell technology.

In the study of iPSCs and ESCs, Oct4 has been identified as a key regulatory gene in reprogramming and inducing cell plasticity (Malik, V et al., Nat. Commun. 2019, 10, 3477). The protein encoded by the Oct4 gene plays a critical role in embryonic development and stem cell pluripotency, with selective splicing leading to various transcript variants. The protein encoded by Oct4 belongs to the POU domain family of transcription factors and is located on Chromosome 17: 35,825,200-35,829,401. The hallmark feature of the POU transcription factor family is the POU domain, which consists of two structurally distinct subdomains: a highly conserved 75-amino-acid POU-specific (POU) region and a 60-amino-acid carboxyl-terminal homologous domain (POUh). The expression of Oct4 is regulated at the transcriptional level by cis-regulatory elements upstream of the Oct4 gene and chromatin structure methylation (Klemm JD, et al., Cell, 1994, 77: 21-32; Brehm A, et al., Mol Cell Biol 1997, 17: 154-162). Yeom and colleagues, by analyzing the expression of a LacZ reporter gene under the control of an 18kb fragment from the Oct4 genomic locus, identified two elements which they named the proximal enhancer (PE) and distal enhancer (DE), where they pinpointed the precise binding sites for transcription factors (Yeom Y, et al., Integrated Ann Indexes 1996; 122: 881-894). POU domain transcription factors bind to specific octamer DNA sequences and regulate cell-type-specific differentiation pathways. During iPSC formation, Oct4, which contains the POU domain, and Sox2, which contains the HMG domain, are essential transcription factors for maintaining pluripotency in pluripotent cells (Nichols, J., et al., Cell, 1998, 95, 379-391; Avilion, A., et al., 2003, Genes Dev. 17, 126-140). Through synergistic interaction between these two transcription factors, the transcription of target genes is driven in pluripotent cells (Tomioka, M., et al., Nucleic Acids Res. 2002; 30, 3202-3213). These findings suggest that developmental transitions can be controlled by Oct4. Currently, widely used reprogramming methods often rely on viruses or other types of vectors to overexpress Oct4 (Takahashi K, et al., Cell, 2006, 126(4): 663-676; Takahashi K and Yamanaka S, Cell, 2007, 131(5): 861-872; Yu J, et al., Science. 2007; 318: 1917-1920). However, these methods present potential clinical risks in the clinical use of iPSCs, such as the tumorigenic risk posed by viral vectors. Additionally, the complex GMP production processes of vectors complicate clinical regulation of iPSCs, and these products often come with high costs due to the use of vectors.

### Summary of the Invention

Currently, compounds that bind to the target protein can be predicted using protein structure prediction tools, and compounds that upregulate the target protein can be obtained through functional screening. Meanwhile, various microRNAs (miRNAs) have been discovered that can downregulate Oct4, showing a strong correlation with the regulation of the complex formed by Sox2 and Nanog. For example, miR-134, miR-145, miR-470, and miR-200c exhibit negative regulatory characteristics on the Oct4, Sox2, and Nanog complex (Esther E. Creemers, Anke J. Tijsen, Yigal M. Pinto, Circ Res, 2012 Feb 3;110(3):483-95). Therefore, inhibitory compounds can also be designed based on the structure of these regulatory miRNAs to inhibit these miRNAs, thereby achieving positive regulation of the Oct4 and Nanog complex.

Based on the above reasons, the present invention designs a pyrrolopyridine derivative that can simultaneously bind to both the Oct4 protein structure and the negative regulatory miRNA structure, based on the structure of the Oct4 protein and the structure of the miRNAs that regulate the Oct4 complex negatively. The pyrrolopyridine derivative can chemically activate Oct4 and regulate the expression of its downstream genes. This avoids the use of viruses or other vectors for Oct4 regulation and further enables safe and simple chemical small molecules to enhance biological expression functions.

The present invention relates to a compound of formula (I) or its pharmaceutically acceptable salts, solvates, active metabolites, polymorphs, esters, optical isomers, or prodrugs, as well as pharmaceutical compositions containing the compound of formula (I) and its use as a high-selectivity Oct4 activator for cell reprogramming.

The present invention provides a compound of formula (I): 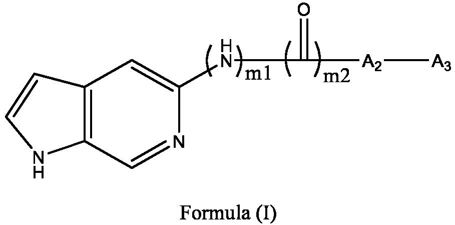 wherein:
m₁ and m₂ are each independently 0 or 1;
A₂ is C₁-C₆ alkylene, C₂-C₆ alkenylene, -O(CH₂)q-, -NR₁-, -SO₂-, -(CH₂)ᵥNHS(O)₂-, or a bond, wherein q is 1, 2, 3, or 4, V is 0, 1, or 2, and R₁ is selected from H or C₁-C₄ alkyl;
A₃ is C₁-C₆ alkyl; C₂-C₆ alkenyl; C₄-C₆ cycloalkyl, wherein one carbon atom may be substituted by a heteroatom selected from N, O, or S; 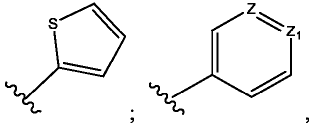 wherein Z and Z₁ are each independently N or CR₂, wherein R₂ is selected from H, halogen, C₁-C₄ alkyl, or cyano; 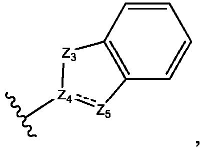 wherein Z₃ is N, O, S, or C=O, when the bond between Z₄ and Z₅ is a single bond, Z₄ is N or CH, and Z₃ is CH₂ or C=O, when the bond between Z₄ and Z₅ is a double bond, Z₄ is C, and Z₅ is CH;
or a pharmaceutically acceptable salt, solvate, active metabolite, polymorph, ester, optical isomer, or prodrug thereof, or a combination thereof.

In some embodiments, A₂ is -CH₂-, -CH=CH-, -C(CH₃)=CH-, -O(CH₂)-, -O(CH₂)₂-, -NH-, -N(CH₃)-, -SO₂-, - NHS(O)₂-, -(CH₂)₂NHS(O)₂- or a bond.

In some embodiments, A₃ is -CH₃, butenyl,

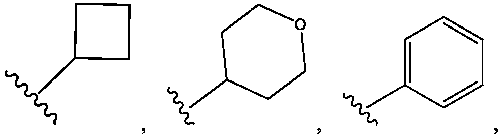

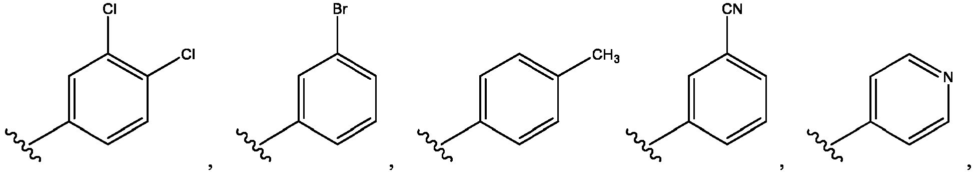

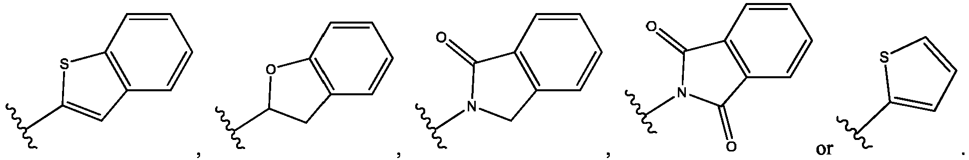

In some embodiments, m1 is 0, m2 is 1; A₂ is -N(CH₃)-; A₃ is

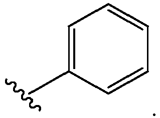

In some embodiments, m1 is 1, m2 is 0; A₂ is -CH₂-, -SO₂-, -(CH₂)₂NHS(O)₂- or a bond; A₃ is -CH₃,

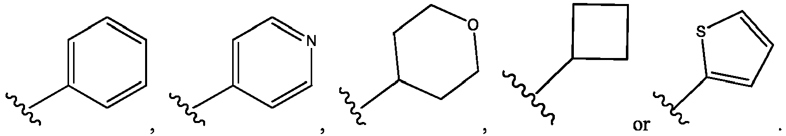

In some embodiments, m1 is 1, m2 is 1; A₂ is -CH₂-, -NH-, -C(CH₃)=CH- or a bond; A₃ is -CH₃, -C(CH₃)=CH-,

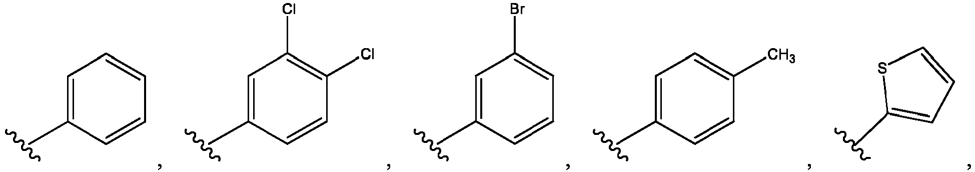

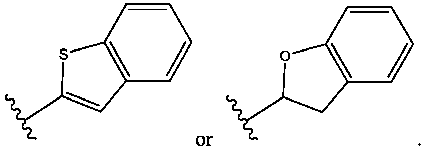

In some embodiments, m1 is 0, m2 is 0; A₂ is -CH₂-, -CH=CH-, -O(CH₂)-, -O(CH₂)₂- or a bond; A₃ is

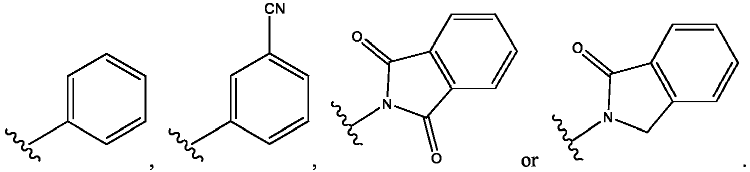

In some embodiments, the compound is selected from:

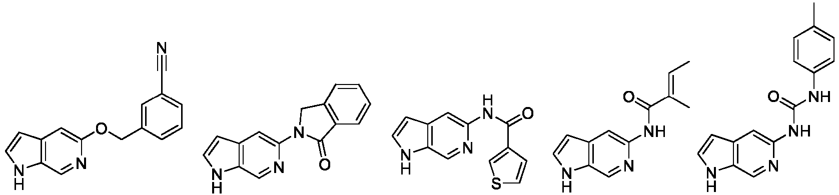

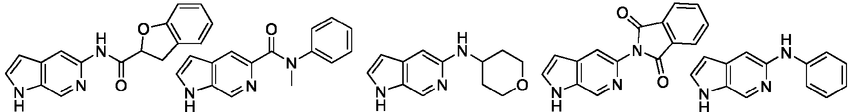

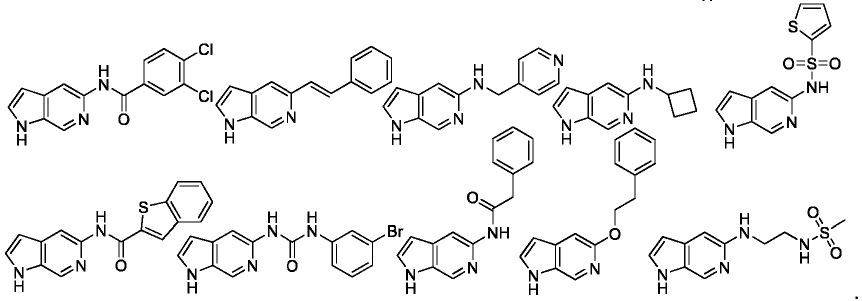

The present invention relates to a pharmaceutical composition, which comprises a compound as described in any of the above, or its pharmaceutically acceptable salts, solvates, active metabolites, polymorphs, esters, optical isomers, or prodrugs, or combinations thereof, and at least one pharmaceutically acceptable carrier or excipient.

The present invention also relates to the use of a compound as described in any of the above, or its pharmaceutically acceptable salts, solvates, active metabolites, polymorphs, esters, optical isomers, or prodrugs, or combinations thereof, in the preparation of an Oct4 high-selectivity activator for inducing pluripotent stem cells.

The present invention also relates to the use of a compound as described in any of the above, or its pharmaceutically acceptable salts, solvates, active metabolites, polymorphs, esters, optical isomers, or prodrugs, or combinations thereof, in the preparation of an Oct4 high-selectivity activator for inducing pluripotent stem cells, wherein the diseases treated by Oct4 high-selectivity activators inducing pluripotent stem cells include at least one of cancer, heart disease, stroke, diabetes, obesity, Alzheimer's disease, Parkinson's disease, amyotrophic lateral sclerosis, myocardial infarction, muscular dystrophy, CMT-1A, spinal cord injury, traumatic brain injury, edentulism, wound healing, bone marrow transplantation, osteoarthritis, rheumatoid arthritis, hair loss, blindness, deafness, Crohn's disease, genetic disorders, and other similar diseases.

The present invention also relates to a method for obtaining induced pluripotent stem cells in a subject suffering from a disease, comprising administering to the subject a therapeutically effective amount of the compound as described in any of the above, or its pharmaceutically acceptable salts, solvates, active metabolites, polymorphs, esters, optical isomers, or prodrugs, or combinations thereof.

In the method of the present invention, a subject suffering from a disease refers to a human suffering from at least one of cancer, heart disease, stroke, diabetes, obesity, Alzheimer's disease, Parkinson's disease, amyotrophic lateral sclerosis, myocardial infarction, muscular dystrophy, CMT-1A, spinal cord injury, traumatic brain injury, edentulism, wound healing, bone marrow transplantation, osteoarthritis, rheumatoid arthritis, hair loss, blindness, deafness, Crohn's disease, genetic disorders, and other similar diseases.

The present invention also relates to a method of activating Oct4, comprising contacting the compound as described in any of the above, or its pharmaceutically acceptable salts, solvates, active metabolites, polymorphs, esters, optical isomers, or prodrugs, or combinations thereof, with the Oct4 target protein.

### Brief Description of the Figures

Figure 1 shows the expression results of the downstream gene Nanog in response to the pyrrolopyridine derivative small molecule (wherein CK represents the control group).
Figure 2 shows the results of the basal expression of Oct4 in response to the pyrrolopyridine derivative small molecule (wherein CK represents the control group).

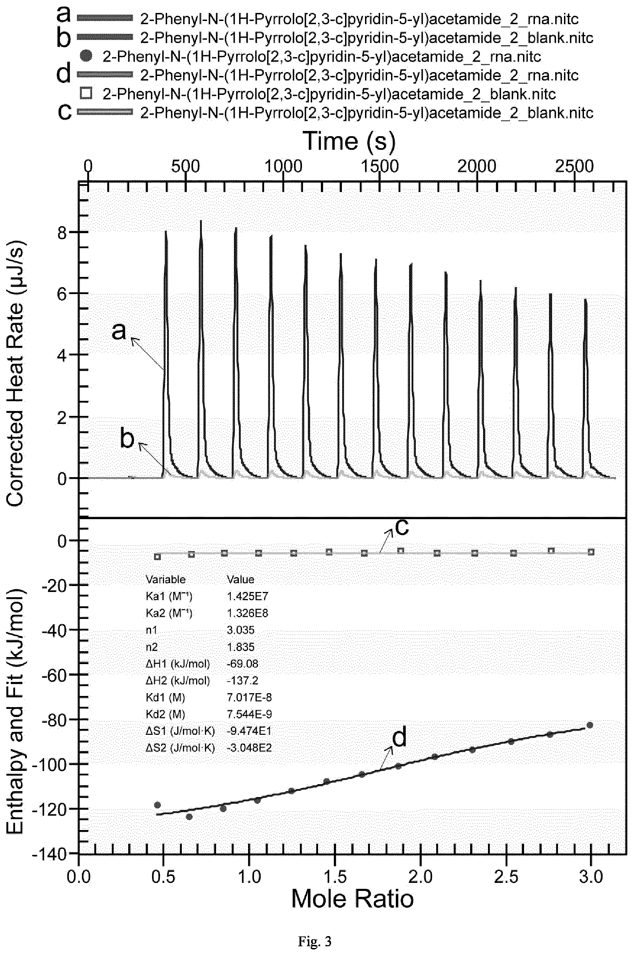

The upper part of Figure 3 shows the corrected heat rate (Corrected Heat Rate, in µJ/s) of the sample pool solution as a function of time (Time, in s). Pulse curve a shows the heat released by the binding of miR-145 and A-3-6 in the sample pool over time (i.e., during the titration process). Pulse curve b shows the heat released by the binding of deionized water and A-3-6 in the sample pool over time (i.e., during the titration process). The lower part of Figure 3 shows curve c, which represents the change in the reaction binding enthalpy (ΔH, in kJ/mol) when A-3-6 solution is titrated into DEPC water (i.e., ultrapure water) that does not contain the nucleic acid miR-145. The change is fitted according to the Multiple Sites model to exclude false-positive heat release due to the dilution of the small molecule during titration. Curve d shows the change in reaction binding enthalpy (ΔH, in kJ/mol) at each drop as the mole ratio of A-3-6 to miR-145 in the titration pool changes. The change is fitted according to the Multiple Sites model.

### Detailed Description of the Invention

In the present invention, the following definitions are applicable:
The term "alkyl" refers to a straight-chain or branched saturated hydrocarbon containing 1-12 carbon atoms. Examples of (C₁-C₆) alkyl groups include but are not limited to methyl, ethyl, propyl, butyl, pentyl, hexyl, isopropyl, isobutyl, sec-butyl, tert-butyl, isopentyl, neopentyl, and isohexyl.

The term "alkenyl" refers to a straight-chain or branched unsaturated hydrocarbon containing 2-12 carbon atoms, with at least one C=C double bond in the chain. Examples of alkenyl groups include vinyl, allyl, butenyl, isobutenyl, pentenyl, or hexenyl.

The term "alkylene" refers to a divalent alkyl group. Any monovalent alkyl group can be converted into an alkylene group by removing a second hydrogen atom from the alkyl. As defined herein, alkylene may also be a C₁-C₆ alkylene. Alkylene may further be a C₁-C₄ alkylene. Typical alkylene groups include but are not limited to: -CH₂-, - CH(CH₃)-, -C(CH₃)₂-, -CH₂CH₂-, -CH₂CH(CH₃)-, -CH2C(CH₃)₂-, -CH₂CH₂CH₂-, -CH₂CH₂CH₂CH₂- and so on.

The term "alkenylene" refers to a divalent alkenyl group. Any monovalent alkenyl group can be converted into an alkenylene group by removing a second hydrogen atom from the alkenyl. As defined herein, alkenylene may further be a C₂-C₆ alkenylene. Typical alkenylene groups include but are not limited to: -CH=CH-, -CH=C(CH₃)-, - CH=CHCH₂-, -CH=CHCH₂CH₂-, -CH=CHCH₂CH₂CH₂-, -CH=CHCH₂CH₂CH₂CH₂- and so on.

The term "cycloalkyl" refers to a monocyclic saturated carbon ring containing 3-18 carbon atoms. Cycloalkyl may further be a C₄-C₆ cycloalkyl group. Examples of cycloalkyl groups include but are not limited to cyclopropyl, cyclobutyl, cyclopentyl, cyclohexyl, cycloheptyl, cyclooctyl, and so on.

The term "halogen" refers to fluorine, chlorine, bromine, or iodine.

The term "cyano" refers to a substituent group consisting of a carbon atom connected to a nitrogen atom through a triple bond (i.e., C≡N).

The term "substitute" as used herein refers to the replacement of one or more hydrogen atoms on a specified atom or group with a group selected from the specified range, provided that the normal valency of the specified atom is not exceeded.

In certain embodiments described in the present invention, the formula (I) compound is provided, wherein when A₂ is "a bond," the structure of the formula (I) compound is:

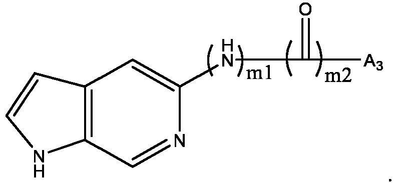

The compounds described herein include, but are not limited to, their optical isomers, racemates, and other mixtures. In such cases, a single enantiomer or diastereomer, i.e., an optically active configuration, can be obtained by asymmetric synthesis or by resolving a racemate or diastereomeric mixture. The resolution of racemates or diastereomeric mixtures can be accomplished by conventional methods, such as crystallization in the presence of a resolving agent or chromatography using, for example, a chiral high-performance liquid chromatography (HPLC) column. Furthermore, these compounds include R- and S-configurations of compounds with chiral centers. These compounds also include crystalline forms, including polymorphs and clathrates. Similarly, the term "salt" also includes all isomers, racemates, other mixtures, R- and S-configurations, tautomers, and crystalline forms of the salts of the described compounds.

The term "pharmaceutically acceptable salt" refers to salts of the compounds represented by formula (I), formula (II), or formula (III) that are non-toxic, biologically tolerable, or otherwise suitable for administration to a subject for therapeutic purposes. See generally: S.M. Berge, et al., "Pharmaceutical Salts," J. Pharm. Sci., 1977, 66: 1-19, and Handbook of Pharmaceutical Salts, Properties, Selection, and Use, Stahl and Wermuth, Eds., Wiley-VCH and VHCA, Zurich, 2002. Preferably, pharmaceutically acceptable salts refer to those that are pharmacologically effective and suitable for contact with patient tissue without undue toxicity, irritation, or allergic response. The compounds of formula (I), formula (II), or formula (III) may possess sufficient acidic groups, basic groups, or both types of functional groups and can form a pharmaceutically acceptable salt by reacting with inorganic or organic bases and inorganic and organic acids. Examples of pharmaceutically acceptable salts include sulfates, pyrosulfates, bisulfates, sulfites, bisulfites, phosphates, monohydrogen phosphates, dihydrogen phosphates, metaphosphates, pyrophosphates, hydrochlorides, hydrobromides, hydroiodides, acetates, propionates, decanoates, octanoates, acrylates, formates, isobutyrates, hexanoates, heptanoates, propargylates, oxalates, malonates, succinates, suberates, sebacates, fumarates, maleates, butyne-1,4-dioates, hexyne-1,6-dioates, benzoates, chlorobenzoates, methylbenzoates, dinitrobenzoates, hydroxybenzoates, methoxybenzoates, phthalates, sulfonates, xylene sulfonates, phenylacetates, phenylpropionates, phenylbutyrates, citrates, lactates, γ-hydroxybutyrates, glycolates, tartrates, methanesulfonates, propanesulfonates, naphthalene-1-sulfonates, naphthalene-2-sulfonates, and mandelates.

A "solvate," such as a "hydrate," is formed through the interaction of a solvent with the compound. The term "compound" includes solvates of the compound, including hydrates. Similarly, "salt" includes solvates of the salt, such as hydrates. Suitable solvates are pharmaceutically acceptable solvates, such as hydrates, including monohydrates and hemihydrates.

A "prodrug" refers to a precursor of the specified compound, which, after administration to a treated subject, is converted to the compound through chemical or physiological processes (such as solvolysis or enzymatic hydrolysis) or under physiological conditions (such as converting the prodrug to the formula (I) compound under physiological pH conditions). A "pharmaceutically acceptable prodrug" is a prodrug that is non-toxic, biologically tolerable, or otherwise biologically suitable for administration to a treated subject. Exemplary procedures for selecting and preparing suitable prodrug derivatives are described in H. Bundgaard's "Design of Prodrugs," Elsevier, 1985.

An "active metabolite" refers to a pharmacologically active product of the metabolism of the formula (I), formula (II), or formula (III) compound or its salt within the body. Prodrugs and active metabolites of compounds can be determined by conventional techniques known or available to those skilled in the art. For example, see Bertolini et al., J. Med. Chem. 1997, 40, 2011-2016; Shan et al., J. Pharm. Sci. 1997, 86(7), 765-767; Bagshawe, Drug Dev. Res. 1995, 34, 220-230; Bodor, Adv. Drug Res. 1984, 13, 224-331; Bundgaard, Design of Prodrugs (Elsevier Press, 1985); and Larsen, Design and Application of Prodrugs, Drug Design and Development (Krogsgaard-Larsen et al., eds., Harwood Academic Publishers, 1991).

A "therapeutically effective amount" refers to an amount of the disclosed compound sufficient to achieve treatment of a disease or condition (as defined below) in a mammal (preferably a human) when administered to the mammal. The amount of the disclosed compound constituting a "therapeutically effective amount" will vary depending on the compound, the condition, the severity of the condition, and the age of the mammal being treated. However, it can be routinely determined by one of ordinary skill in the art using their own knowledge and the teachings herein.

The term "treat" or "treatment" refers to the administration of a compound described herein, or its pharmaceutically acceptable salt, solvate, active metabolite, polymorph, ester, optical isomer, or prodrug, or a combination thereof, preferably at least one compound disclosed herein and/or at least one of its pharmaceutically acceptable salts, to mitigate (reduce) undesirable physiological changes or diseases, such as the development or spread of inflammation or cancer. Beneficial or desired clinical outcomes of the present invention include, but are not limited to: alleviation of symptoms, reduction in the severity of the disease, stabilization (i.e., prevention from worsening) of the disease state, delay or slowing of disease progression, improvement or relief of the condition, and remission (whether partial or complete) of the disease, whether detectable or undetectable. "Treatment" also means extending the lifespan compared to the expected lifespan of those not receiving treatment. Individuals in need of treatment include those exhibiting symptoms of or suffering from such diseases.

### Pharmaceutical Composition

The present invention provides a pharmaceutical composition comprising the compounds described herein, or their pharmaceutically acceptable salts, solvates, active metabolites, polymorphs, esters, optical isomers, or prodrugs, or combinations thereof as active ingredients. Preferably, one or more of the compounds described herein or their pharmaceutically acceptable salts or esters are used as active ingredients; and one or more pharmaceutically acceptable excipients or carriers, including inert solid diluents and fillers, diluents including sterile aqueous solutions and various organic solvents, permeation enhancers, solubilizers, and adjuvants. The pharmaceutical composition can be administered alone or in combination with other therapeutic agents. Such compositions are prepared in a manner known in the field of pharmaceutics.

The pharmaceutical composition can be administered as a single dose or multiple doses, via any acceptable method for agents with similar purposes, such as those described in the patents and patent applications referenced herein, including rectal, buccal, nasal, and transdermal routes, by intra-arterial injection, intravenous, intraperitoneal, parenteral, intramuscular, subcutaneous, oral, topical, as an inhalant, or through implantation or coating devices such as stents, for example, or arterial insertion of cylindrical polymers.

The present invention also provides a kit comprising a pharmaceutical composition containing the compound of the invention or its pharmaceutically acceptable salt, and at least one pharmaceutically acceptable carrier.

"Pharmaceutically acceptable carrier or excipient" refers to a non-toxic, biologically tolerable, and otherwise biologically suitable substance for administration to a subject, such as an inert substance, which is added to a pharmaceutical composition or used as a medium, carrier, or diluent to facilitate the administration of the active ingredient and is compatible therewith. Examples of excipients include calcium carbonate, calcium phosphate, various types of sugars or starches, cellulose derivatives, gelatin, vegetable oils, and polyethylene glycol.

"Combination" should be understood to mean the combination of two or more of the following: a compound, its pharmaceutically acceptable salt, its pharmaceutically acceptable solvate, its pharmaceutically acceptable active metabolite, its pharmaceutically acceptable polymorph, its pharmaceutically acceptable ester, its pharmaceutically acceptable optical isomer, and its pharmaceutically acceptable prodrug.

### Uses of Compounds and Their Compositions

The present invention provides the use of a compound and its compositions primarily as an Oct4-selective activator for activating Oct4 function. By chemically regulating the Oct4 promoter, it achieves the expression regulation of downstream genes, thereby inducing pluripotent stem cells in subjects with diseases to achieve therapeutic purposes. The diseases include cancer, heart disease, stroke, diabetes, obesity, Alzheimer's disease, Parkinson's disease, amyotrophic lateral sclerosis, myocardial infarction, muscular dystrophy, CMT-1A, spinal cord injury, traumatic brain injury, tooth loss, wound healing, bone marrow transplantation, osteoarthritis, rheumatoid arthritis, hair loss, blindness, deafness, Crohn's disease, genetic disorders, and other similar diseases.

### List of abbreviations used in the following examples and elsewhere herein:

CH₂Cl₂: Dichloromethane; Cs₂CO₃: Cesium carbonate; Cu₂SO₄: Copper(II) sulfate; DCM: Dichloromethane; DMAC: N,N-Dimethylacetamide; DMF: N,N-Dimethylformamide; DIEA: N,N-Diisopropylethylamine; DIOX: 1,4-Dioxane; EA: Acetic acid; Et₃N: Triethylamine; ETOH: Ethanol; EtOAc: Ethyl acetate; FA: Formic acid; g: Gram; h: Hour; HATU: 2-(7-Azabenzotriazol-1-yl)-N,N,N',N'-tetramethyluronium hexafluorophosphate; HBr: Hydrobromic acid; (Hbim)BF₄: 1-Butylimidazolium tetrafluoroborate; H₂O: Water; HAc: Acetic acid; H₂O₂: Hydrogen peroxide; H₂SO₄: Fuming sulfuric acid; KCN: Potassium cyanide; K₂CO₃: Potassium carbonate; MCA: Chloroacetic acid; MeCN: Acetonitrile; MeOH: Methanol; mg: Milligram; mL: Milliliter; mmol: Millimole; mol: Mole; mol/L: Mole per liter; m/z: Mass-to-charge ratio; N₂: Nitrogen; NaBH₃CN: Sodium cyanoborohydride; NaNO₂: Sodium nitrite; NaOH: Sodium hydroxide; Na₂SO₄: Sodium sulfate; PH: pH; TBN: tert-Butyl nitrite; TEA: Triethanolamine; THF: Tetrahydrofuran; TLC: Thin-layer chromatography; µL: Microliter; Xylene: Xylene.

The following embodiments and drawings further illustrate the technical means adopted by the present invention to achieve the intended purpose. Unless otherwise specified, the experimental methods used in the following examples are conventional methods. The raw materials, reagents, and other materials used in the following examples are commercially available products unless otherwise specified.

### General Synthesis:

The general synthetic routes described in this application can be modified by substituting the starting materials with other materials of similar structure, thereby obtaining different products accordingly. The following descriptions of synthetic routes provide multiple examples of how the starting materials can be varied to obtain corresponding products.

### General Method A-1-n

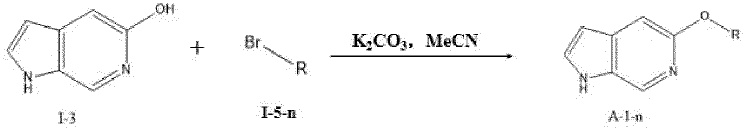

Wherein R is 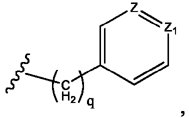 wherein q is 1, 2, 3, or 4, Z and Z₁ are each independently N or CR₂, and R₂ is selected from H, halogen, C₁-C₄ alkyl, or cyano.

### General Method A-3-n

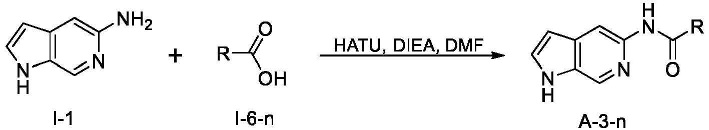

Wherein R is C₂-C₆ alkenyl; 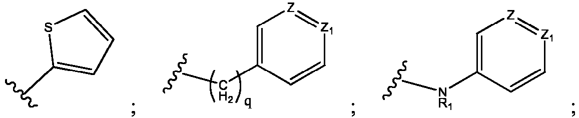 or 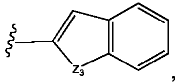 wherein q is 0, 1, 2, 3, or 4, Z and Z₁ are each independently N or CR₂, R₂ is selected from H, halogen, C₁-C₄ alkyl, or cyano, R₁ is selected from H or C₁-C₄ alkyl, and Z₃ is N, O, S, or C=O.

### General Method A-4-n

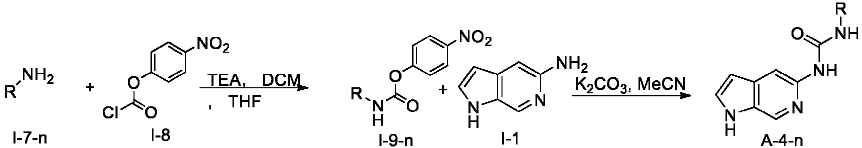

Wherein R is 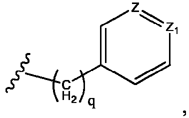 wherein q is 0, 1, 2, 3, or 4, Z and Z₁ are each independently N or CR₂, and R₂ is selected from H, halogen, C₁-C₄ alkyl, or cyano.

### General Method A-6-n

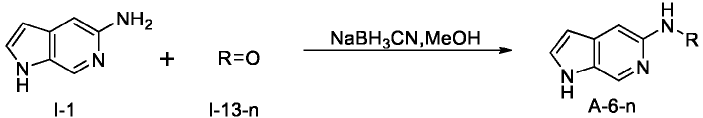

Wherein R is a C₄-C₆ cycloalkyl group, wherein one carbon atom may be substituted by a heteroatom selected from N, O, or S; or R=O is 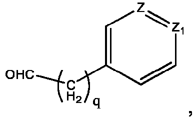 wherein q is 0, 1, 2, 3, or 4, Z and Z₁ are each independently N or CR₂, and R₂ is selected from H, halogen, C₁-C₄ alkyl, or cyano.

### Intermediate Synthesis

### Intermediate I-3: 1H-Pyrrolo[2,3-c]pyridine-5-ol

### Synthesis route:

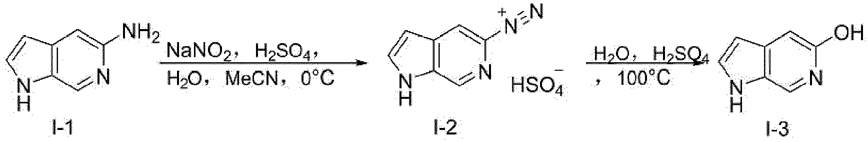

### Step 1: Intermediate 1H-Pyrrolo[2,3-c]pyridine-5-diazonium I-2

To 1H-pyrrolo[2,3-c]pyridine-5-amine 1-1 (66.6 mg, 0.5 mmol), add water (2 mL) and 20% H₂SO₄ aqueous solution (1 mL). Under ice-cooling, add sodium nitrite aqueous solution (42 mg, 0.6 mmol) (0.5 mL) and acetonitrile (2 mL) to the mixture, and stir for 30 minutes. To the resulting reaction mixture, add a solution of Cu₂SO₄ (67 mg, 0.3 mmol) in 20% HBr aqueous solution (0.5 mL) at room temperature, and stir the mixture at 80 °C for 30 minutes. Add ethyl acetate and water to the reaction mixture. Separate the organic layer, wash with brine, dry with sodium sulfate, and concentrate under reduced pressure. The residue is purified by silica gel column chromatography (chloroform: methanol = 50:1) to obtain 1H-pyrrolo[2,3-c]pyridine-5-diazonium I-2 hydrogen sulfate salt (94 mg, 78%), with liquid chromatography mass spectrometry m/z = 145.1 [M]⁺.

### Step 2: Intermediate 1H-Pyrrolo[2,3-c]pyridine-5-ol I-3

Add a solution (1 mL) of the hydrogen sulfate salt of 1H-pyrrolo[2,3-c]pyridine-5-diazonium I-2 (48 mg, 0.2 mmol) to 40% sulfuric acid aqueous solution (5 mL) at 100 °C, and stir the mixture for 10 minutes. Add NaOH to the resulting reaction mixture to bring the pH to about 3, then add ethyl acetate. Separate the organic layer, wash with brine, dry with sodium sulfate, and concentrate under reduced pressure. The residue is purified by silica gel column chromatography (chloroform: methanol = 50:1) to obtain 1H-pyrrolo[2,3-c]pyridine-5-ol I-3 (20 mg, 77%), with liquid chromatography mass spectrometry m/z = 135.1 [M+H]⁺.

### Example 1: ((1H-Pyrrolo[2,3-c]pyridine-5-yl)oxy)methyl)benzonitrile A-1-1

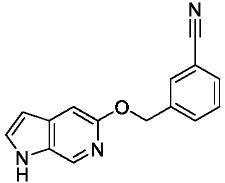

Step: Mix 1H-pyrrolo[2,3-c]pyridine-5-ol I-3 (10 mg, 0.07 mmol) with 3-(bromomethyl)benzonitrile (20 mg, 0.1 mmol) and K₂CO₃ (138 mg, 1 mmol), add acetonitrile (2 mL), and heat the mixture at 40 °C while stirring for 10 minutes. Add ethyl acetate and water to the reaction mixture, separate the organic layer, wash with sodium sulfate, and concentrate under reduced pressure. The residue is purified by silica gel column chromatography (dichloromethane: methanol = 40:1) to obtain 3-((1H-pyrrolo[2,3-c]pyridine-5-yl)oxy)methyl)benzonitrile A-1-1 (11 mg, 63%), with liquid chromatography mass spectrometry m/z = 250.1 [M+H]⁺.

### Example 2: 5-Phenylethoxy-1H-pyrrolo[2,3-c]pyridine A-1-2

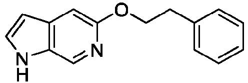

Step: Mix 1H-pyrrolo[2,3-c]pyridine-5-ol I-3 (10 mg, 0.07 mmol) with (2-bromoethyl)benzene (22 mg, 0.12 mmol) and K₂CO₃ (138 mg, 1 mmol), add acetonitrile (2 mL), and heat the mixture to 40 °C while stirring for 10 minutes. Add ethyl acetate and water to the reaction mixture, separate the organic layer, wash with sodium sulfate, and concentrate under reduced pressure. The residue is purified by silica gel column chromatography (dichloromethane: methanol = 40:1) to obtain 5-phenylethoxy-1H-pyrrolo[2,3-c]pyridine A-1-2 (13 mg, 78%), with liquid chromatography mass spectrometry m/z = 239.1 [M+H]⁺.

### Example 3: (1H-pyrrolo[2,3-c]pyridine-5-yl) isoindole-1-one A-2

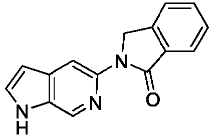

### Synthesis Route:

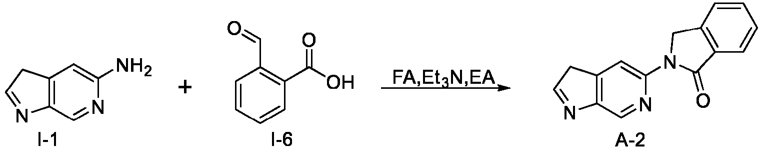

Step: Mix 1H-pyrrolo[2,3-c]pyridine-5-amine I-1 (13 mg, 0.1 mmol) with 2-formylbenzoic acid (18 mg, 0.12 mmol), add formic acid (0.2 mL), triethylamine (1 mL), and ethanol (1 mL), and heat the mixture to 80 °C while stirring for 60 minutes. Add ethyl acetate and water to the reaction mixture, separate the organic layer, wash with sodium sulfate, and concentrate under reduced pressure. The residue is purified by silica gel column chromatography (dichloromethane: ethyl acetate = 20:1) to obtain (1H-pyrrolo[2,3-c]pyridine-5-yl)isoindole-1-one A-2 (15 mg, 60%), with liquid chromatography mass spectrometry m/z = 250.3 [M+H]⁺.

### Example 4: (1H-pyrrolo[2,3-c]pyridine-5-yl) thieno[3,2-b]pyridine-3-carboxamide A-3-1

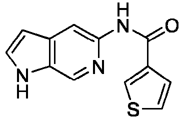

Step: Mix 1H-pyrrolo[2,3-c]pyridine-5-amine I-1 (13 mg, 0.1 mmol) with thieno[3,2-b]pyridine-2-carboxylic acid (15 mg, 0.12 mmol) and 2-(7-aza-benzotriazole)-N,N,N',N'-tetramethylurea hexafluorophosphate (38 mg, 0.1 mmol), add N,N-diisopropylethylamine (0.2 mL), N,N-dimethylformamide (2 mL), and stir at room temperature for 18 hours. Add ethyl acetate and water to the reaction mixture, separate the organic layer, wash with sodium sulfate, and concentrate under reduced pressure. The residue is purified by silica gel column chromatography (ethyl acetate: hexane = 3:7) to obtain N-(1H-pyrrolo[2,3-c]pyridine-5-yl)thieno[3,2-b]pyridine-3-carboxamide A-3-1 (10 mg, 41%), with liquid chromatography mass spectrometry m/z = 244.1 [M+H]⁺.

### Example 5: (E)-2-Methyl-N-(1H-pyrrolo[2,3-c]pyridine-5-yl)but-2-enamide A-3-2

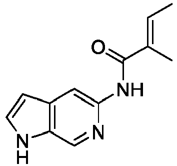

Step: Mix 1H-pyrrolo[2,3-c]pyridine-5-amine I-1 (13 mg, 0.1 mmol) with (E)-2-methyl-2-enecarboxylic acid (12 mg, 0.12 mmol) and 2-(7-aza-benzotriazole)-N,N,N',N'-tetramethylurea hexafluorophosphate (38 mg, 0.1 mmol), add N,N-diisopropylethylamine (0.2 mL), N,N-dimethylformamide (2 mL), and stir at room temperature for 18 hours. Add ethyl acetate and water to the reaction mixture, separate the organic layer, wash with sodium sulfate, and concentrate under reduced pressure. The residue is purified by silica gel column chromatography (ethyl acetate: hexane = 3:7) to obtain (E)-2-methyl-N-(1H-pyrrolo[2,3-c]pyridine-5-yl)but-2-enamide A-3-2 (11 mg, 51%), with liquid chromatography mass spectrometry m/z = 216.1 [M+H]⁺.

### Example 6: N-(1H-pyrrolo[2,3-c]pyridine-5-yl)-2,3-dihydrobenzo[b]furan-2-carboxamide A-3-3

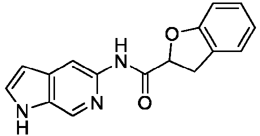

Step: Mix 1H-pyrrolo[2,3-c]pyridine-5-amine 1-1 (13 mg, 0.1 mmol) with benzo[b]furan-2-carboxylic acid (19 mg, 0.12 mmol) and 2-(7-aza-benzotriazole)-N,N,N',N'-tetramethylurea hexafluorophosphate (38 mg, 0.1 mmol), add N,N-diisopropylethylamine (0.2 mL), N,N-dimethylformamide (2 mL), and stir at room temperature for 18 hours. Add ethyl acetate and water to the reaction mixture, separate the organic layer, wash with sodium sulfate, and concentrate under reduced pressure. The residue is purified by silica gel column chromatography (ethyl acetate: hexane = 3:7) to obtain N-(1H-pyrrolo[2,3-c]pyridine-5-yl)-2,3-dihydrobenzo[b]furan-2-carboxamide A-3-3 (13 mg, 46%), with liquid chromatography mass spectrometry m/z = 280.1 [M+H]⁺.

### Example 7: 3,4-Dichloro-N-(1H-Pyrrolo[2,3-c]pyridin-5-yl)benzamide A-3-4

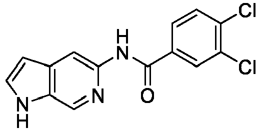

Step: 1H-Pyrrolo[2,3-c]pyridin-5-amine I-1 (13 mg, 0.1 mmol) was mixed with 3,4-dichlorobenzoic acid (23 mg, 0.12 mmol) and 2-(7-Nitrobenzotriazolyl)-N,N,N',N'-tetramethylurea hexafluorophosphate (38 mg, 0.1 mmol), then N,N-Diisopropylethylamine (0.2 mL) and N,N-Dimethylformamide (2 mL) were added. The mixture was stirred at room temperature for 18 hours. Ethyl acetate and water were added to the reaction mixture, and the organic layer was separated, dried over sodium sulfate, and concentrated under reduced pressure. The residue was purified by silica gel column chromatography (ethyl acetate: hexane = 3:7), yielding 3,4-dichloro-N-(1H-Pyrrolo[2,3-c]pyridin-5-yl)benzamide A-3-4 (13 mg, 43%) with liquid chromatography-mass spectrometry (LC-MS) m/z = 306.0 [M+H]⁺.

### Example 8: N-(1H-Pyrrolo[2,3-c]pyridin-5-yl)benzothiophene-2-carboxamide A-3-5

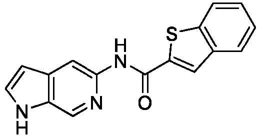

Step: 1H-Pyrrolo[2,3-c]pyridin-5-amine 1-1 (13 mg, 0.1 mmol) was mixed with benzothiophene-2-carboxylic acid (21 mg, 0.12 mmol) and 2-(7-Nitrobenzotriazolyl)-N,N,N',N'-tetramethylurea hexafluorophosphate (38 mg, 0.1 mmol), then N,N-Diisopropylethylamine (0.2 mL) and N,N-Dimethylformamide (2 mL) were added. The mixture was stirred at room temperature for 18 hours. Ethyl acetate and water were added to the reaction mixture, and the organic layer was separated, dried over sodium sulfate, and concentrated under reduced pressure. The residue was purified by silica gel column chromatography (ethyl acetate: hexane = 3:7), yielding N-(1H-Pyrrolo[2,3-c]pyridin-5-yl)benzothiophene-2-carboxamide A-3-5 (14 mg, 48%) with LC-MS m/z = 294.1 [M+H]⁺.

### Example 9: 2-Phenyl-N-(1H-Pyrrolo[2,3-c]pyridin-5-yl)acetamide A-3-6

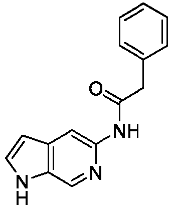

Step: 1H-Pyrrolo[2,3-c]pyridin-5-amine I-1 (13 mg, 0.1 mmol) was mixed with 2-phenylacetic acid (16 mg, 0.12 mmol) and 2-(7-Nitrobenzotriazolyl)-N,N,N',N'-tetramethylurea hexafluorophosphate (38 mg, 0.1 mmol), then N,N-Diisopropylethylamine (0.2 mL) and N,N-Dimethylformamide (2 mL) were added. The mixture was stirred at room temperature for 18 hours. Ethyl acetate and water were added to the reaction mixture, and the organic layer was separated, dried over sodium sulfate, and concentrated under reduced pressure. The residue was purified by silica gel column chromatography (ethyl acetate: hexane = 3:7), yielding 2-phenyl-N-(1H-Pyrrolo[2,3-c]pyridin-5-yl)acetamide A-3-6 (12 mg, 48%) with LC-MS m/z = 252.1 [M+H]⁺.

### Example 10: 1-(1H-Pyrrolo[2,3-c]pyridin-5-yl)-3-(p-Tolyl)urea A-4-1

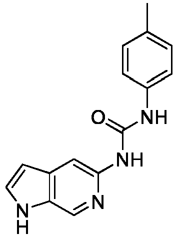

### Step 1: p-Nitrobenzyl-p-tolylcarbamate I-9-1

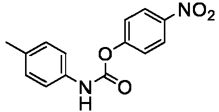

p-Toluidine (21 mg, 0.2 mmol) was mixed with 4-nitrobenzyl chloroformate (48 mg, 0.24 mmol), and triethanolamine (0.2 mL), dichloromethane (1 mL), and tetrahydrofuran (2 mL) were added. The mixture was stirred at room temperature for 18 hours. Ethyl acetate and water were added to the reaction mixture, and the organic layer was separated, dried over sodium sulfate, and concentrated under reduced pressure. The residue was purified by silica gel column chromatography (dichloromethane: hexane = 5:1), yielding p-nitrobenzyl-p-tolylcarbamate 1-9-1 (42 mg, 77%) with LC-MS m/z = 273.1 [M+H]⁺.

### Step 2: 1-(1H-Pyrrolo[2,3-c]pyridin-5-yl)-3-(p-Tolyl)urea A-4-1

The above p-nitrobenzyl-p-tolylcarbamate 1-9-1 (27 mg, 0.1 mmol) was mixed with 1H-Pyrrolo[2,3-c]pyridin-5-amine I-1 (16 mg, 0.12 mmol) and K₂CO₃ (0.2 g, 1.45 mmol), then acetonitrile (3 mL) was added, and the mixture was heated to 40 °C and stirred for 4 hours. After the reaction was complete (monitored by TLC), the reaction mixture was concentrated under vacuum. The crude reaction mixture was washed with dichloromethane, then with EtOAc, and finally with MeOH (each 1 mL). The reaction product was recrystallized from EtOAc (under warm conditions), yielding 1-(1H-Pyrrolo[2,3-c]pyridin-5-yl)-3-(p-Tolyl)urea A-4-1 (22 mg, 83%) with LC-MS m/z = 267.1 [M+H]⁺.

### Example 11: 1-(3-Bromophenyl)-3-(1H-Pyrrolo[2,3-c]pyridin-5-yl)urea A-4-2

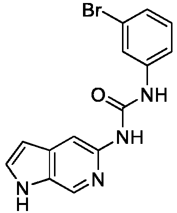

### Step 1: 4-Nitrobenzyl(3-bromophenyl)carbamate 1-9-2

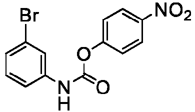

3-Bromoaniline (34 mg, 0.2 mmol) was mixed with 4-nitrobenzyl chloroformate (48 mg, 0.24 mmol), and triethanolamine (0.2 mL), dichloromethane (1 mL), and tetrahydrofuran (2 mL) were added. The mixture was stirred at room temperature for 18 hours. Ethyl acetate and water were added to the reaction mixture, and the organic layer was separated, dried over sodium sulfate, and concentrated under reduced pressure. The residue was purified by silica gel column chromatography (dichloromethane: hexane = 5:1), yielding 4-nitrobenzyl(3-bromophenyl)carbamate 1-9-2 (46 mg, 68%) with LC-MS m/z = 338.1 [M+H]⁺.

### Step 2: 1-(3-Bromophenyl)-3-(1H-Pyrrolo[2,3-c]pyridin-5-yl)urea A-4-2

The above 4-nitrobenzyl(3-bromophenyl)carbamate 1-9-2 (34 mg, 0.1 mmol) was mixed with 1H-Pyrrolo[2,3-c]pyridin-5-amine 1-1 (16 mg, 0.12 mmol) and K₂CO₃ (0.2 g, 1.45 mmol), then acetonitrile (3 mL) was added, and the mixture was heated to 40 °C and stirred for 4 hours. After the reaction was complete (monitored by TLC), the reaction mixture was concentrated under vacuum. The crude reaction mixture was washed with dichloromethane, then with EtOAc, and finally with MeOH (each 1 mL). The reaction product was recrystallized from EtOAc (under warm conditions), yielding 1-(3-Bromophenyl)-3-(1H-Pyrrolo[2,3-c]pyridin-5-yl)urea A-4-2 (25 mg, 75%) with LC-MS m/z = 331.2 [M+H]⁺.

### Example 12: N-Methyl-N-phenyl-1H-Pyrrolo[2,3-c]pyridin-5-carboxamide A-5

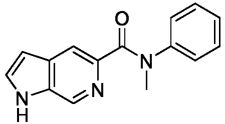

### Synthesis route:

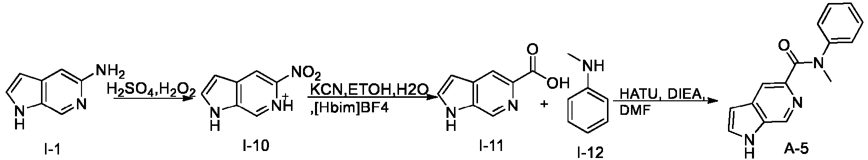

### Step 1: 5-Nitro-1H-Pyrrolo[2,3-c]pyridine 1-10

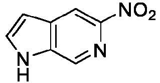

A solution of H2O2 (240 mg, 2.2 mmol) in fuming sulfuric acid (500 µL) was added dropwise to a solution of 1H-Pyrrolo[2,3-c]pyridin-5-amine 1-1 (40 mg, 0.3 mmol) in concentrated sulfuric acid (100 µL), and the reaction temperature was maintained at 0 °C. After stirring at 10 °C -25 °C for 3 hours, the reaction mixture was adjusted to pH 11-12 by adding 40% NaOH solution at 0 °C -5 °C. The resulting mixture was extracted with ethyl acetate. The organic layer was washed with saturated sodium chloride solution, dried over Na₂SO₄, and filtered. The solvent was removed under reduced pressure to yield the desired 5-nitro-1H-Pyrrolo[2,3-c]pyridine I-10 (39 mg, 80%) with LC-MS m/z = 164.0 [M+H]⁺.

### Step 2: 1H-Pyrrolo[2,3-c]pyridin-5-carboxylic acid I-11

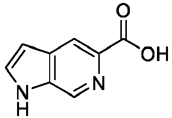

The above 5-nitro-1H-Pyrrolo[2,3-c]pyridine I-10 (39 mg, 0.24 mmol) was mixed with KCN (195 mg, 3 mmol) and 1-butylimidazole tetrafluoroborate (3 mg, 0.014 mmol), then EtOH (2 mL) and water (2 mL) were added and heated to 80 °C for 19 hours. Afterward, 10 mL of water was added, and the mixture was extracted with CH₂Cl₂ (3×5 mL) and ether (3×10 mL). The mixture was acidified with hydrochloric acid to pH 1-2, and extracted with ether (3×10 mL). Magnesium sulfate (3 g) and activated charcoal (1 g) were added and stirred for 5 hours. The solid was filtered off, and the filtrate was evaporated, and the residue was crystallized from the corresponding solvent, yielding the desired 1H-Pyrrolo[2,3-c]pyridin-5-carboxylic acid I-11 (16 mg, 41%) with LC-MS m/z = 163.0 [M+H]⁺.

### Step 3: N-Methyl-N-phenyl-1H-Pyrrolo[2,3-c]pyridin-5-carboxamide A-5

The above 1H-Pyrrolo[2,3-c]pyridin-5-carboxylic acid I-11 (16 mg, 0.1 mmol) was mixed with N-methyl aniline (13 mg, 0.12 mmol) and 2-(7-Nitrobenzotriazolyl)-N,N,N',N'-tetramethylurea hexafluorophosphate (38 mg, 0.1 mmol), then N,N-Diisopropylethylamine (0.2 mL) and N,N-Dimethylformamide (2 mL) were added. The mixture was stirred at room temperature for 18 hours. Ethyl acetate and water were added to the reaction mixture, and the organic layer was separated, dried over sodium sulfate, and concentrated under reduced pressure. The residue was purified by silica gel column chromatography (ethyl acetate: hexane = 3:7), and the solvent was removed by rotary evaporation, yielding N-Methyl-N-phenyl-1H-Pyrrolo[2,3-c]pyridin-5-carboxamide A-5 (18 mg, 72%) with LC-MS m/z = 252.1 [M+H]⁺.

### Example 13: N-(Tetrahydro-2H-pyran-4-yl)-1H-pyrrolo[2,3-c]pyridine-5-amine A-6-1

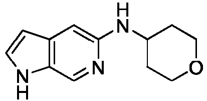

Mix 1H-pyrrolo[2,3-c]pyridine-5-amine I-1 (13 mg, 0.1 mmol) with tetrahydro-4H-pyran-4-one (12 mg, 0.12 mmol), add methanol (2 mL), and stir at room temperature for 2 hours. Then add sodium cyanoborohydride (20 mg, 0.3 mmol) and stir at room temperature for 2 hours. Add NaOH aqueous solution (10 mL, 0.3 mol/L) to the mixture. Extract the resulting mixture with DCM (20 mL × 3). Combine the organic phases, dry over anhydrous Na₂SO₄, filter, and concentrate under vacuum. Purify the crude product by silica gel column chromatography (petroleum ether: ethyl acetate = 5:1), and remove the solvent by rotary evaporation to obtain N-(tetrahydro-2H-pyran-4-yl)-1H-pyrrolo[2,3-c]pyridine-5-amine A-6-1 (18 mg, 83%), with liquid chromatography-mass spectrometry (LC-MS) m/z = 218.1 [M+H]⁺.

### Example 14: N-(Pyridine-4-ylmethyl)-1H-pyrrolo[2,3-c]pyridine-5-amine A-6-2

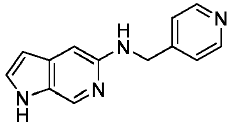

Mix 1H-pyrrolo[2,3-c]pyridine-5-amine I-1 (13 mg, 0.1 mmol) with isonicotinaldehyde (13 mg, 0.12 mmol), add methanol (2 mL), and stir at room temperature for 2 hours. Then add sodium cyanoborohydride (20 mg, 0.3 mmol) and stir at room temperature for 2 hours. Add NaOH aqueous solution (10 mL, 0.3 mol/L) to the mixture. Extract the resulting mixture with DCM (20 mL × 3). Combine the organic phases, dry over anhydrous Na₂SO₄, filter, and concentrate under vacuum. Purify the crude product by silica gel column chromatography (petroleum ether: ethyl acetate = 5:1), and remove the solvent by rotary evaporation to obtain N-(pyridine-4-ylmethyl)-1H-pyrrolo[2,3-c]pyridine-5-amine A-6-2 (16 mg, 71%), with LC-MS m/z = 225.1 [M+H]⁺.

### Example 15: N-Cyclobutyl-1H-pyrrolo[2,3-c]pyridine-5-amine A-6-3

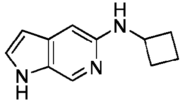

Mix 1H-pyrrolo[2,3-c]pyridine-5-amine I-1 (13 mg, 0.1 mmol) with cyclobutanone (8 mg, 0.12 mmol), add methanol (2 mL), and stir at room temperature for 2 hours. Then add sodium cyanoborohydride (20 mg, 0.3 mmol) and stir at room temperature for 2 hours. Add NaOH aqueous solution (10 mL, 0.3 mol/L) to the mixture. Extract the resulting mixture with DCM (20 mL × 3). Combine the organic phases, dry over anhydrous Na₂SO₄, filter, and concentrate under vacuum. Purify the crude product by silica gel column chromatography (petroleum ether: ethyl acetate = 5:1), and remove the solvent by rotary evaporation to obtain N-cyclobutyl-1H-pyrrolo[2,3-c]pyridine-5-amine A-6-3 (15 mg, 80%), with LC-MS m/z = 188.1 [M+H]⁺.

### Example 16: 2-(1H-pyrrolo[2,3-c]pyridine-5-yl)isoindole-1,3-dione A-7

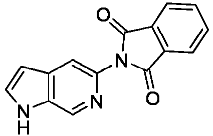

### Synthesis route:

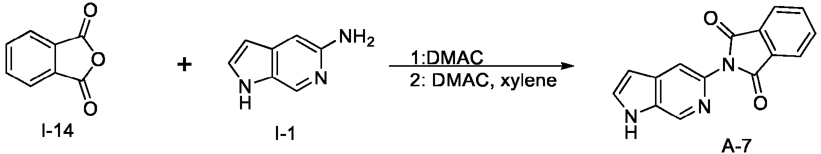

Step: Mix 1H-pyrrolo[2,3-c]pyridine-5-amine I-1 (13 mg, 0.1 mmol) with isoindole-1,3-dione (18 mg, 0.12 mmol), add N,N-dimethylacetamide (2 mL), and stir at room temperature for 24 hours. Then add xylene (1 mL) and stir at 140 °C in an oil bath for 48 hours. After completion (monitored by TLC), filter to separate the insoluble catalyst, wash with acetone, and dry. Concentrate the organic phase under reduced pressure to obtain the desired product. Wash with water and recrystallize from ethanol. Purify the crude product by silica gel column chromatography (CH2C12/hexane = 1:1), and remove the solvent by rotary evaporation to obtain 2-(1H-pyrrolo[2,3-c]pyridine-5-yl)isoindole-1,3-dione A-7 (20 mg, 76%), with LC-MS m/z = 264.1 [M+H]⁺.

### Example 17: N-Phenyl-1H-pyrrolo[2,3-c]pyridine-5-amine A-8

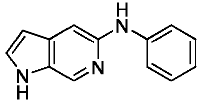

### Synthesis route:

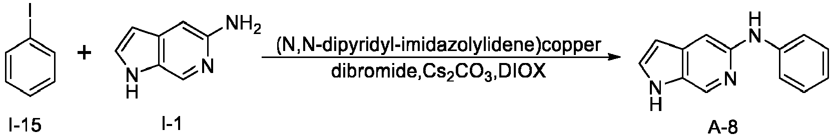

Step: Mix 1H-pyrrolo[2,3-c]pyridine-5-amine I-1 (13 mg, 0.1 mmol) with iodobenzene (24 mg, 0.12 mmol), (N,N-bipyridyl-imidazolyl)dibromocopper (10 mg, 0.023 mmol), and cesium carbonate (100 mg, 0.3 mmol), add 1,4-dioxane (5 mL), and stir at 170 °C in an oil bath for 12 hours. Then add NaOH aqueous solution (10 mL, 0.3 mol/L) to the mixture. Extract the resulting mixture with DCM (20 mL × 3). Combine the organic phases, dry over anhydrous Na₂SO₄, filter, and concentrate under vacuum. Purify the crude product by silica gel column chromatography (petroleum ether: ethyl acetate = 5:1), and remove the solvent by rotary evaporation to obtain N-phenyl-1H-pyrrolo[2,3-c]pyridine-5-amine A-8 (19 mg, 91%), with LC-MS m/z = 210.1 [M+H]⁺.

### Example 18: (E)-5-Styryl-1H-pyrrolo[2,3-c]pyridine A-9

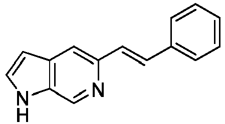

### Synthesis route:

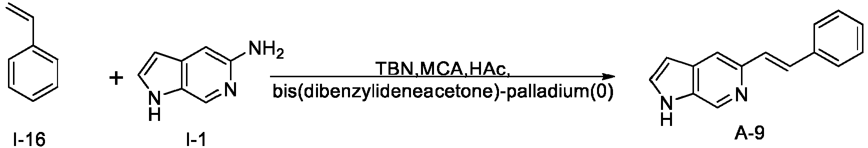

Step: Mix 1H-pyrrolo[2,3-c]pyridine-5-amine I-1 (13 mg, 0.1 mmol) with styrene (13 mg, 0.12 mmol) and bis(dibenzylideneacetone)palladium(0) (5 mg, 0.0087 mmol), add tert-butyl nitrite (0.5 mL), chloroacetic acid (0.5 mL), and acetic acid (3 mL), and stir at 50 °C in an oil bath for 2 hours. Then add NaOH aqueous solution (10 mL, 0.3 mol/L) to the mixture. Extract the resulting mixture with DCM (20 mL × 3). Combine the organic phases, dry over anhydrous Na2SO4, filter, and concentrate under vacuum. Purify the crude product by silica gel column chromatography (petroleum ether: ethyl acetate = 5:1), and remove the solvent by rotary evaporation to obtain (E)-5-styryl-1H-pyrrolo[2,3-c]pyridine A-9 (15 mg, 68%), with LC-MS m/z = 221.1 [M+H]⁺.

### Example 19: N-(1H-pyrrolo[2,3-c]pyridine-5-yl)thiophene-2-sulfonamide A-10

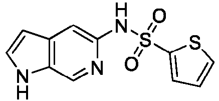

### Synthesis route:

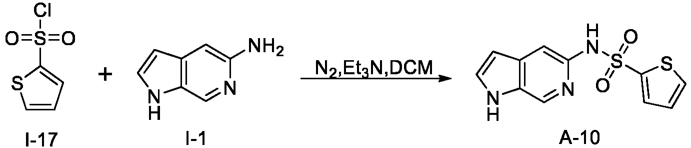

Step: Mix 1H-pyrrolo[2,3-c]pyridine-5-amine I-1 (13 mg, 0.1 mmol) with thiophene-2-sulfonyl chloride (22 mg, 0.12 mmol), add triethylamine (0.5 mL), dichloromethane (3 mL), and stir at room temperature for 24 hours under nitrogen protection. Add ethyl acetate and water to the reaction mixture, separate the organic phase, dry with sodium sulfate, and concentrate under reduced pressure. Purify the residue by silica gel column chromatography (ethyl acetate: hexane = 3:7), and remove the solvent by rotary evaporation to obtain N-(1H-pyrrolo[2,3-c]pyridine-5-yl)thiophene-2-sulfonamide A-10 (14 mg, 50%), with LC-MS m/z = 280.0 [M+H]⁺.

### Example 20: N-(2-((1H-pyrrolo[2,3-c]pyridine-5-yl)amino)ethyl)methanesulfonamide A-11

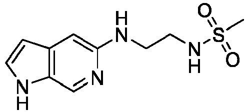

### Synthesis route:

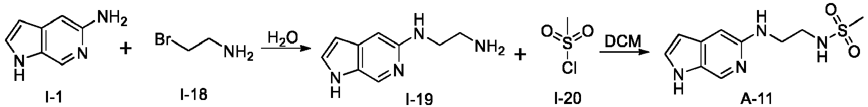

### Step 1: N1-(1H-pyrrolo[2,3-c]pyridine-5-yl)ethane-1,2-diamine I-19

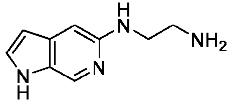

Mix 1H-pyrrolo[2,3-c]pyridine-5-amine 1-1 (26 mg, 0.2 mmol) with 2-bromoethane-1-amine I-18 (29 mg, 0.24 mmol), add water (5 mL), and stir at 95°C in an oil bath for 18 hours. Add ethyl acetate and water to the reaction mixture, separate the organic phase, dry with sodium sulfate, and concentrate under reduced pressure. Purify the mixture by flash chromatography on silica gel (CH2Cl2 methanol- ammonia) to obtain the desired product. Remove the solvent by rotary evaporation to obtain N1-(1H-pyrrolo[2,3-c]pyridine-5-yl)ethane-1,2-diamine I-19 (24 mg, 68%), with LC-MS m/z = 177.1 [M+H]⁺.

### Step 2: N-(2-((1H-pyrrolo[2,3-c]pyridine-5-yl)amino)ethyl)methanesulfonamide A-11

Mix the above N1-(1H-pyrrolo[2,3-c]pyridine-5-yl)ethane-1,2-diamine I-19 (24 mg, 0.13 mmol) with methanesulfonyl chloride 1-20 (28 mg, 0.16 mmol), add dichloromethane (5 mL), and stir at 0°C for 24 hours. Add ethyl acetate and water to the reaction mixture, separate the organic phase, dry with sodium sulfate, and concentrate under reduced pressure. Purify the residue by silica gel column chromatography (ethyl acetate: hexane = 3:7), and remove the solvent by rotary evaporation to obtain N-(2-((1H-pyrrolo[2,3-c]pyridine-5-yl)amino)ethyl)methanesulfonamide A-11 (12 mg, 36%), with LC-MS m/z = 255.1 [M+H]⁺.

### Small molecule binding energy prediction:

In this invention, two compound prediction methods are used to predict the binding of pyrrolopyridine derivatives, as described below:

### Method 1: Protein structure docking prediction

AutoDock Vina and LeDock software were used to perform molecular docking between the pyrrolopyridine derivatives from examples 1-20 and the Oct4 target protein, generating 10 docking conformations for each compound. The binding energy and ligand efficiency of each pyrrolopyridine molecule with the Oct4 target protein were calculated based on the optimal docking results. The docking results were comprehensively used for screening pyrrolopyridine molecules. The specific docking results are shown in Table 1: The second and third columns of the table show the binding free energy (binding energy) obtained from AutoDock Vina and LeDock docking software, respectively. The more negative the value, the stronger the binding ability between the small molecule ligand and the target protein. The fourth column represents the ligand efficiency calculated by LeDock, where a larger absolute value indicates a stronger potential for the small molecule's activity. In this invention, based on the independent algorithms of the two software programs, the binding energy levels of the compounds involved in this invention were predicted, and the predicted values show that the binding energies of the compounds in this invention are all significantly greater than the threshold value of 3 set based on the target characteristics.

**Table 1. Binding energy prediction of pyrrolopyridine derivatives with the target sequence used in this invention.**

| Compound | AD_vina_binding_energy (kcal/mol) | Ledock_Energy (kcal/mol) | Ledock_Ligand_efficiency |
|---|---|---|---|
| A-1-1 | -6.2 | -5.05 | -0.27 |
| A-1-2 | -5.9 | -4.92 | -0.27 |
| A-2 | -6.4 | -4.50 | -0.24 |
| A-3-1 | -5.3 | -4.78 | -0.28 |
| A-3-2 | -5.6 | -5.03 | -0.31 |
| A-3-3 | -6.4 | -4.68 | -0.22 |
| A-3-4 | -6.5 | -4.85 | -0.24 |
| A-3-5 | -6.6 | -4.85 | -0.23 |
| A-3-6 | -6 | -4.81 | -0.25 |
| A-4-1 | -6 | -4.80 | -0.24 |
| A-4-2 | -5.9 | -5.24 | -0.26 |
| A-5 | -6 | -4.26 | -0.22 |
| A-6-1 | -5.6 | -4.54 | -0.28 |
| A-6-2 | -5.8 | -4.95 | -0.29 |
| A-6-3 | -5.3 | -4.39 | -0.31 |
| A-7 | -6.7 | -4.67 | -0.23 |
| A-8 | -5.85 | -4.57 | -0.29 |
| A-9 | -6.2 | -4.70 | -0.28 |
| A-10 | -5.3 | -5.36 | -0.30 |
| A-11 | -4.85 | -5.62 | -0.33 |

### Method 2: miR-RNA structure docking prediction:

The prediction of the affinity between miR-145 and small molecules was carried out using a new computational tool, RLDOCK software. RLDOCK is a ligand-RNA binding affinity prediction model based on physical deformation. It employs a novel global multi-step search algorithm for binding sites, optimizing search efficiency and stability, allowing the algorithm to effectively predict unknown binding sites. In the RLDOCK multi-step calculation, potential binding sites on the miR-145 structure and possible binding orientations of the small molecule at those sites are first scanned in detail. A minimal Lennard-Jones potential energy scoring function is used to assign scores to all potential binding poses and rank them. The results are shown in Table 2: The scoring result is a negative value, and the larger the absolute value, the stronger the ligand-RNA affinity. The final score of RLDOCK is the total value from various energy functions it employs. Currently, the number of validated RNA targets is limited, and there is no mature computational system that can distinguish the threshold for actual interactions. This method prioritizes the verification of high-ranking molecules based on predicted binding energy.

**Table 2: Binding energy prediction of pyrrolopyridine derivatives with the target miR-RNA.**

| Compound | RLDOCK_SF-h_SCORE(Based_on_Energy) |
|---|---|
| A-1-1 | -16.35 |
| A-1-2 | -22.13 |
| A-2 | -14.17 |
| A-3-1 | -17.16 |
| A-3-2 | -17.55 |
| A-3-3 | -11.27 |
| A-3-4 | -15.99 |
| A-3-5 | -6.05 |
| A-3-6 | -18.14 |
| A-4-1 | -20.06 |
| A-4-2 | -17.58 |
| A-5 | -17.28 |
| A-6-1 | -16.28 |
| A-6-2 | -20.83 |
| A-6-3 | -1.24 |
| A-7 | -5.41 |
| A-8 | -19.77 |
| A-9 | -12.25 |
| A-10 | -18.08 |
| A-11 | -17.71 |

Through Oct4 protein structure docking prediction and miR-145 structure docking prediction, it was found that pyrrolopyridine derivatives exhibited significant binding energies in both structural docking predictions. Therefore, it is speculated that pyrrolopyridine derivatives enhance the expression of Oct4. To verify this, isothermal titration calorimetry (ITC) and cellular experiments were conducted to confirm the effect of pyrrolopyridine derivatives on the enhancement of Oct4 expression, thereby validating the high selectivity activation effect of pyrrolopyridine derivatives on Oct4.

### Using isothermal titration calorimetry to verify the interaction of small molecules with the target nucleic acid:

Isothermal titration calorimetry (ITC) utilizes the principle of power compensation to accurately obtain the enthalpy change of intermolecular interactions within a broad concentration range in the system through a single concentration scan. It measures the heat change during binding and provides the complete thermodynamic characteristics of nucleic acid-ligand interactions. Quantifying the thermodynamic properties and energy of the complex is essential for analyzing the molecular basis of the interaction between the microRNA miR-145 and the compound. The present invention uses the Waters Nano-ITC calorimeter to in vitro verify the nucleic acid-small molecule interactions.

The experimental process involves placing 300µL of a 50µM single-stranded miR-145 solution in a temperaturecontrolled sample chamber, and using small molecule A-3-6 (i.e., the 2-phenyl-N-(1H-pyrrolo[2,3-c]pyridine-5-yl)acetamide obtained in Example 9) as the ligand in a 50µL, 500µM concentration, which is gradually injected (or titrated) into the sample chamber. The heat change is measured by comparing the sample chamber with a reference chamber, showing either an endothermic or exothermic peak. The interaction pattern between the two reactants is fitted using the software provided by the instrument, and it directly calculates the reaction binding enthalpy (ΔH, kJ/mol), reaction binding entropy (ΔS, J/(mol·K)), number of binding sites (n), and binding equilibrium constant (Ka, M), obtaining the kinetic data. The upper part of Figure 3 shows the corrected heat power change of the solution in the sample chamber over time. Pulse curve (a) shows the exothermic binding of miR-145 with A-3-6 over time (i.e., as titration proceeds). Pulse curve (b) shows the exothermic binding of DEPC water (blank control) with A-3-6 over time. The lower part of Figure 3 shows curve (c), which represents the change in reaction binding enthalpy (ΔH, kJ/mol) when titrating DEPC water without miR-145 with A-3-6, fitted using the Multiple Sites model to exclude false positive heat release from small molecule dilution during titration; curve (d) shows the change in reaction binding enthalpy (ΔH) with the change in molar ratio of A-3-6 to miR-145, fitted using the Multiple Sites model. Since the blank control does not contain small nucleic acid molecules (miR-145), the slope of curve (c) only reflects the heat release stability during small molecule titration of the blank control. The calculated number of binding sites (nSite) for A-3-6 with miR-145 solution are n1=3 and n2=1.8, with Kd1=7.017E-8 and Kd2=7.544E-9, ΔH1=-69.08 and ΔH2=-137.2, ΔS1=-9.474E1 and ΔS2=-3.048E2. The ITC experimental results indicate that the small molecule A-3-6 obtained in the present invention can specifically bind to the target single-stranded miR-145.

### Verification of transcriptional expression differences caused by small molecules:

The aim of the present invention is to use highly selective activators to enhance the expression of target genes. An important function of such activators is to increase the expression of Oct4, thereby increasing the expression abundance of downstream genes regulated by Oct4. Therefore, when verifying the function of the small molecules in the present invention, in addition to verifying the increase in the expression of Oct4 itself, the increase in the expression of Nanog, a downstream gene of Oct4, is also an indicator for functional verification of the compounds in the present invention.

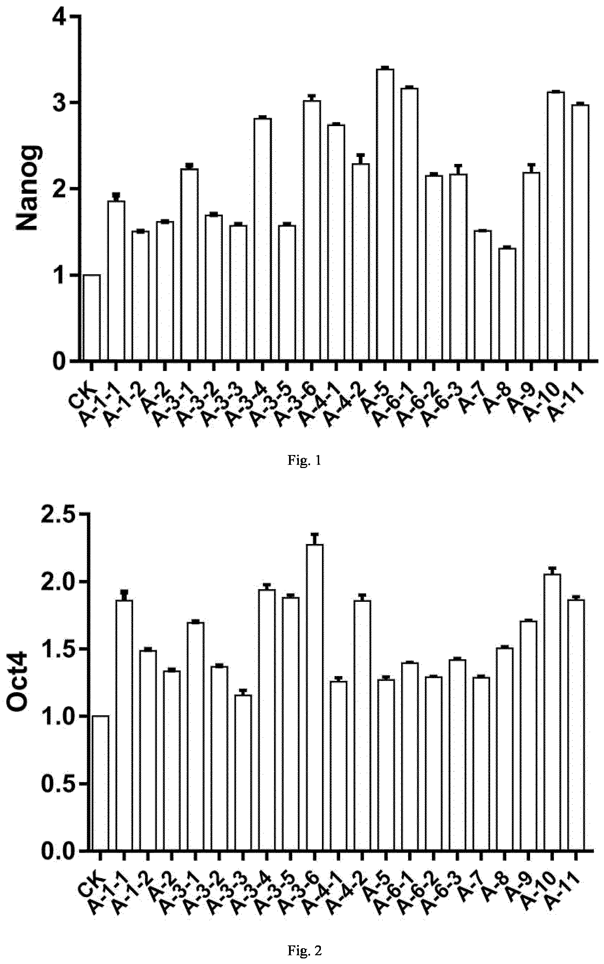

Human mesenchymal stem cells were cultured in T25 flasks at a density of 4x10^5 cells, using serum-free Dulbecco's modified Eagle medium (DMEM-F 12 medium), with 50nM of the above pyrrolopyridine derivative small molecules added for culture. The culture conditions were 37°C and 5% CO2. On day 5, total RNA was extracted using the RNeasy Mini or Micro Kit (QIAGEN), and 1 mg RNA was used to synthesize cDNA using the SuperScript III First-Strand Synthesis System (Invitrogen). Quantitative PCR was performed using SYBR Premix Ex Taq (TaKaRa) and the Thermal Cycler Dice Real-Time System (TaKaRa), with beta-actin as the internal reference. All data were analyzed using the delta-Ct method. Three replicate experiments were performed for each group, and variance statistics were conducted. The primer sequences for identifying different cell marker genes are shown in Table 3. The results are shown in Figures 1 and 2, where, compared with the control group without small molecules, the pyrrolopyridine derivative small molecules significantly increased the baseline expression of Oct4 and its downstream gene Nanog.

**Table 3: Primer sequences for QPCR of compound effect genes.**

| | |
|---|---|
| Oct4-F | CCATGCATTCAAACTGAGGT |
| Oct4-R | CCTTTGTGTTCCCAATTCCTT |
| Nanog-F | ACCTCAGCTACAAACAGGTGAA |
| Nanog-R | AAAGGCTGGGGTAGGTAGGT |
| βActin-F | GGCCGAGGACTTTGATTGCACA |
| βActin-R | GGGCACGAAGGCTCATCATTCAA |

## Claims

1. A compound of formula (I): 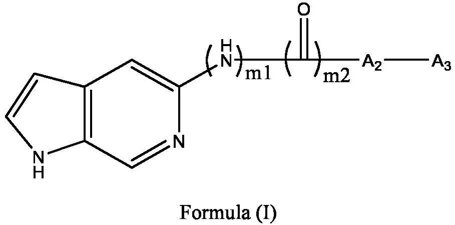 wherein:
m₁ and m₂ are each independently 0 or 1;
A₂ is C₁-C₆ alkylene, C₂-C₆ alkenylene, -O(CH₂)q-, -NR₁-, -SO₂-, -(CH₂)_{V}NHS(O)₂-, or a bond, wherein q is 1, 2, 3, or 4, V is 0, 1, or 2, and R₁ is selected from H or C₁-C₄ alkyl;
A₃ is C₁-C₆ alkyl; C₂-C₆ alkenyl; C₄-C₆ cycloalkyl, wherein one carbon atom may be substituted by a heteroatom selected from N, O, or S; 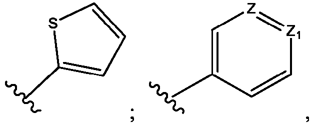 wherein Z and Z₁ are each independently N or CR₂, wherein R₂ is selected from H, halogen, C₁-C₄ alkyl, or cyano; 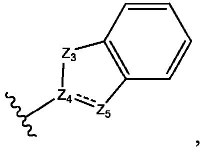 wherein Z₃ is N, O, S, or C=O, when the bond between Z₄ and Z₅ is a single bond, Z₄ is N or CH, and Z₅ is CH₂ or C=O, when the bond between Z₄ and Z₅ is a double bond, Z₄ is C, and Z₅ is CH;
or a pharmaceutically acceptable salt, solvate, active metabolite, polymorph, ester, optical isomer, or prodrug thereof, or a combination thereof.

2. The compound according to claim 1, wherein:
A₂ is -CH₂-, -CH=CH-, -C(CH₃)=CH-, -O(CH₂)-, -O(CH₂)₂-, -NH-, -N(CH₃)-, -SO₂-, -NHS(O)₂-, - (CH₂)₂NHS(O)₂- or a bond.

3. The compound according to claim 2, wherein:
A₃ is -CH₃, butenyl,

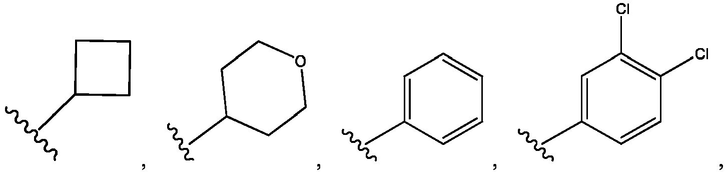

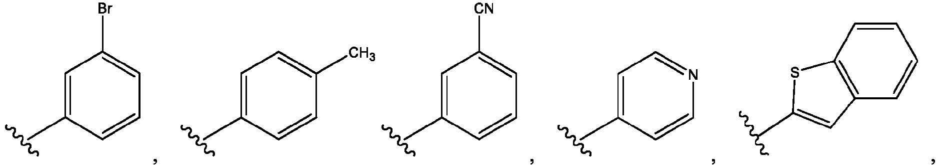

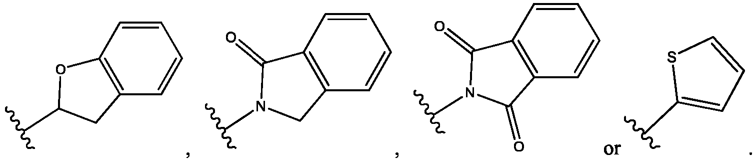

4. The compound according to claim 3, wherein:
m1 is 0, m2 is 1;
A₂ is -N(CH₃)-;
A3 is

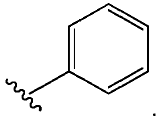

5. The compound according to claim 3, wherein:
m1 is 1, m2 is 0;
A₂ is -CH₂-, -SO₂-, -(CH₂)₂NHS(O)₂- or a bond;
A₃ is -CH₃,

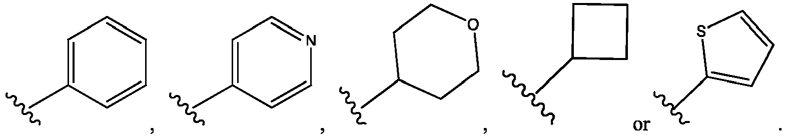

6. The compound according to claim 3, wherein:
m1 is 1, m2 is 1;
A₂ is -CH₂-, -NH-, -C(CH₃)=CH- or a bond;
A₃ is -CH₃, -C(CH₃)=CH-,

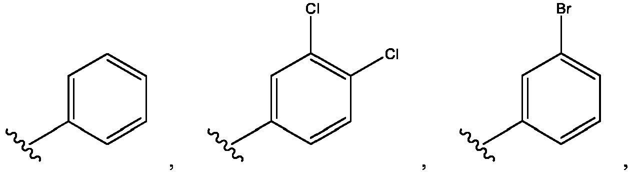

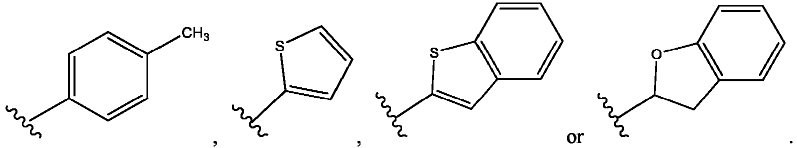

7. The compound according to claim 3, wherein:
m1 is 0, m2 is 0;
A₂ is -CH₂-, -CH=CH-, -O(CH₂)-, -O(CH₂)₂- or a bond;
A₃ is

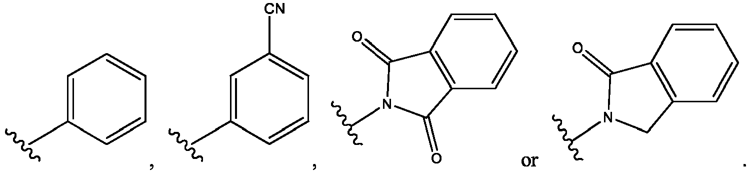

8. The compound according to claim 3, wherein the compound is selected from:

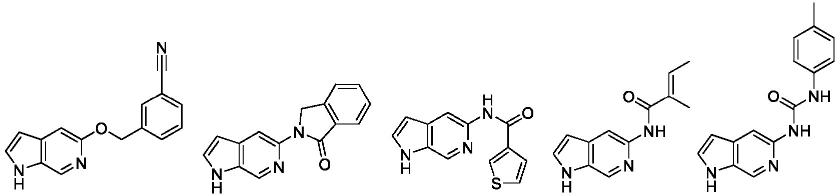

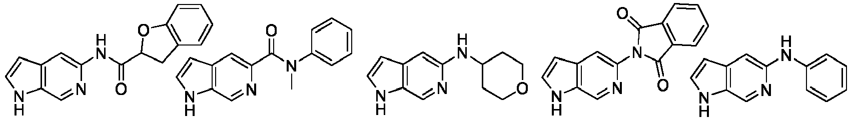

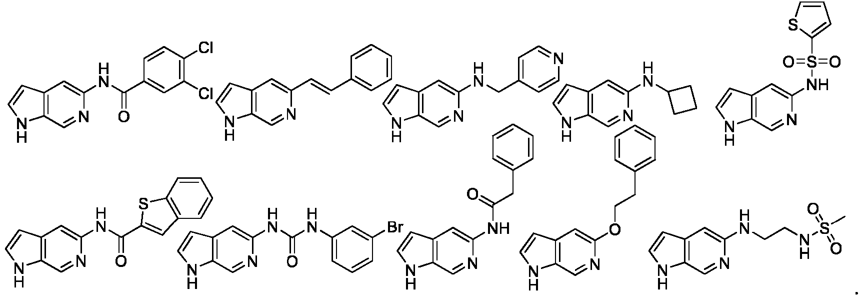

9. A pharmaceutical composition comprising: a compound according to any one of claims 1 to 8, or a pharmaceutically acceptable salt, solvate, active metabolite, polymorph, ester, optical isomer, or prodrug thereof, or a combination thereof, and at least one pharmaceutically acceptable carrier or excipient.

10. Use of a compound according to any one of claims 1 to 8, or a pharmaceutically acceptable salt, solvate, active metabolite, polymorph, ester, optical isomer, or prodrug thereof, or a combination thereof, in the preparation of an Oct4 highly selective activator for inducing pluripotent stem cells.

11. The use according to claim 10, wherein the diseases treated by the Oct4 highly selective activator-induced pluripotent stem cells include at least one of cancer, heart disease, stroke, diabetes, obesity, Alzheimer's disease, Parkinson's disease, amyotrophic lateral sclerosis, myocardial infarction, muscular dystrophy, CMT-1A, spinal cord injury, traumatic brain injury, tooth loss, wound healing, bone marrow transplantation, osteoarthritis, rheumatoid arthritis, hair loss, blindness, deafness, Crohn's disease, genetic disorders, and other similar diseases.

12. A method for obtaining induced pluripotent stem cells in a subject suffering from a disease, comprising administering to the subject a therapeutically effective amount of a compound according to any one of claims 1 to 8, or a pharmaceutically acceptable salt, solvate, active metabolite, polymorph, ester, optical isomer, or prodrug thereof, or a combination thereof.

13. The method according to claim 12, wherein the subject suffering from a disease refers to a human suffering from cancer, heart disease, stroke, diabetes, obesity, Alzheimer's disease, Parkinson's disease, amyotrophic lateral sclerosis, myocardial infarction, muscular dystrophy, CMT-1A, spinal cord injury, traumatic brain injury, tooth loss, wound healing, bone marrow transplantation, osteoarthritis, rheumatoid arthritis, hair loss, blindness, deafness, Crohn's disease, genetic disorders, and other similar diseases.

14. A method for activating Oct4, comprising contacting an Oct4 target protein with a compound according to any one of claims 1 to 8, or a pharmaceutically acceptable salt, solvate, active metabolite, polymorph, ester, optical isomer, or prodrug thereof, or a combination thereof.
